# EUROPEAN PATENT SPECIFICATION

(11) **EP 0 880 488 B1**
(45) Date of publication and mention of the grant of the patent: **12.12.2001**
(21) Application number: 97901600.3
(22) Date of filing: 22.01.1997
(51) Int. Cl.: C07C 15/04, C07C 15/08

(54) **PROCESS FOR CONVERSION OF ETHYLBENZENE**
VERFAHREN ZUR UMSETZUNG VON ETHYLBENZOL
PROCEDE POUR LA CONVERSION DE L'ETHYLBENZENE

(30) Priority: 25.01.1996 US 591064
(43) Date of publication of application: 02.12.1998
(73) Proprietor: ExxonMobil Chemical Patents Inc., Linden, NJ 07036-0710 (US)
(72) Inventor: CLEM, Kenneth, Ray, Humble, TX 77346 (US); LATTNER, James, Richardson, Seabrook, TX 77586 (US)
(74) Representative: White, Nicholas John, Dr.
(86) International application number: PCT/EP97/00317
(87) International publication number: WO 97/27161

(56) References cited:
- WO-A-89/09642
- WO-A-96/01686
- US-A- 3 504 048

## Description

This invention relates to a process and a chemical plant for the conversion of ethylbenzene. In particular it is directed to use of molecular sieve membranes for enhanced recovery and conversion of ethylbenzene.

In the petrochemical production chain some of the most important streams are those which comprise aromatic components and in particular C₆ to C₈ aromatic components; these being a valuable source of raw materials for high value downstream products. From such streams, benzene, toluene and the C₈ aromatics which are particularly valuable may be obtained. The C₈ aromatics are orthoxylene, metaxylene, paraxylene and ethylbenzene. Paraxylene is often the most desirable of the xylenes; however because the boiling points of ethylbenzene, ortho-, meta- and paraxylene (hereinafter collectively referred to as "C₈ aromatics") are close, they are difficult to separate by fractional distillation. As a consequence various alternative methods of separating paraxylene and the other C₈ aromatics from streams comprising aromatics have been developed.
The most common of such methods are fractional crystallisation which utilises the difference in freezing points between ethylbenzene, ortho-, meta- and paraxylene, and selective adsorption which commonly utilises zeolite materials to selectively adsorb paraxylene from C₈ aromatics streams; the adsorbed paraxylene is recovered after desorbing from the zeolite. When either of these processes are used paraxylene can be recovered in high yields from the C₈ aromatics stream. The resulting filtrate from the crystallisation process or the raffinate from the adsorption process are depleted in paraxylene and contain relatively high proportions of ethylbenzene, ortho-,and metaxylene. These streams are typically. subjected to further processing downstream of the crystallisation or adsorption process.

Typically for a paraxylene loop one of the additional downstream processes is an isomerisation process which is used to increase the proportion of paraxylene in paraxylene depleted streams from such processes as fractional crystallisation or selective adsorption. The xylenes, which are predominately ortho-and metaxylene, can be contacted with an isomerisation catalyst under appropriate temperature and pressure which results in the conversion of some of the ortho-and metaxylene to paraxylene. It is also usually necessary to convert most of the ethylbenzene to prevent it from building up to high concentrations in the loop but to also obtain more useful aromatics such as paraxylene and benzene. A catalyst can be selected to enable conversion of ethylbenzene to benzene, and/or to orthoxylene and metaxylene through a C₈ naphthene intermediate and/or to C₁₀ aromatics and benzene via transalkylation. It may be that the catalyst for conversion of ethylbenzene to orthoxylene is also a xylenes isomerisation catalyst in which case the orthoxylene from the ethylbenzene is converted to an equilibrium mixture of xylenes.

Such prior art processes for making paraxylene have typically included combinations of isomerization with fractional crystallisation and/or adsorption separation. The problem with this combination is that despite improvements in catalyst performance the isomerisation technology is still relatively inefficient for the conversion of ethylbenzene to benzene or xylenes and only produces equilibrium or near-equilibrium mixtures of xylenes . The consequence of this is that big recycles of the xylenes stream back through these processes are needed to ensure the conversion of the C₈ aromatics stream to paraxylene is maximised with or without the additional recovery if desired of orthoxylene and/or metaxylene. Furthermore the xylenes isomerization unit is usually operated under relatively severe conditions, such as high temperature, for the xylenes isomerisation process in order to ensure adequate conversion of the ethylbenzene to useful product. Such severe operating conditions result in significant xylenes losses in the xylenes isomerisation unit. Also there are a number of streams in the petrochemical production chain which comprise aromatics and which are not normally used for feeds to a paraxylene recovery process. Such streams may have high concentrations of ethylbenzene e.g. pyrolysis gasoline C₈ streams which could be a valuable source of other aromatics such as paraxylene. These streams are usually fed into the motor gasoline pool.

There is a need therefore for improved processes and chemical plants for the conversion of ethylbenzene containing streams e.g. C₈ aromatics streams, which in particular address the problems associated with large recycles and in particular low ethylbenzene conversions.

Zeolite membranes have been described in the prior art, for example in US 4,699,892, US 5,100,596, EP 0481658, EP 0481659, EP 0481660, WO 92/13631, WO 93/00155, WO 94/01209, and WO 94/25151. However the prior art does not describe how to use such membranes in actual ethylbenzene conversion processes in the petrochemical cycle nor does the prior art describe how to use such membranes in combination with existing processes to significantly enhance the production of paraxylene from ethylbenzene containing streams.

The present invention is therefore directed to a chemical plant and process which offers an improvement over the prior art for the conversion of ethylbenzene and in particular the conversion of ethylbenzene from a C₈ aromatics stream into paraxylene. The present invention resides in the specific application of a molecular sieve membrane unit and process in an ethylbenzene conversion process. This invention utilises molecular sieve membranes to continuously separate ethylbenzene or an ethylbenzene/paraxylene mixture from a feed; the ethylbenzene may be converted to xylenes and/or benzene. The use of a molecular sieve membrane unit and process in such a conversion process provides for a significant improvement in ethylbenzene conversion. Also when this is part of a xylenes loop also provides for enhanced paraxylene production compared to conventional paraxylene recovery processes.

The present invention therefore provides a process for conversion of ethylbenzene to benzene and/or xylenes which process comprises:
(a) passing at least a portion of a stream comprising ethylbenzene to a molecular sieve membrane unit comprising a molecular sieve membrane, for a time and under such conditions that at least a portion of the ethylbenzene passes through the molecular sieve membrane to the permeate side of the membrane and
(b) converting the ethylbenzene in at least a portion of said permeate to benzene and/or xylenes.

In one embodiment the ethylbenzene conversion process is part of a xylene recovery loop. In this embodiment it is preferred that the feed to the molecular sieve membrane unit is from a paraxylene recovery process and comprises the paraxylene depleted stream from such process which is enriched in ethylbenzene when compared to the feed to the paraxylene recovery process. In this embodiment it is also preferred that the ethylbenzene conversion process is undertaken in an ethylbenzene conversion unit which is downstream of the molecular sieve membrane unit and that at least a portion of the permeate from the molecular sieve membrane unit is fed to the ethylbenzene conversion unit. The ethylbenzene conversion unit produces a stream which is depleted in ethylbenzene when compared to the permeate from the molecular sieve membrane unit and which is enriched in ethylbenzene conversion products such as benzene and/or xylenes. In this embodiment it is also preferred that the conversion stream from the ethylbenzene conversion unit, optionally combined with the retentate from the molecular sieve membrane unit, is subjected to a xylenes isomerisation process in a second isomerisation unit to produce an isomerate stream having an enriched paraxylene content compared to the feed to the isomerisation unit and feeding the isomerate stream to a paraxylene recovery process.

Preferably there is a further molecular sieve membrane unit which is located downstream of the xylenes isomerisation unit and which produces a permeate enriched in paraxylene compared to the isomerate. Preferably this further molecular sieve membrane unit additionally comprises a xylenes isomerisation catalyst.

In a second embodiment there is present an ethylbenzene conversion catalyst either as part of the molecular sieve membrane or downstream of the molecular sieve membrane but in close proximity to the membrane or both. Most preferably the isomerisation catalyst in the molecular sieve membrane unit is located in close proximity to the membrane and on the permeate side of the membrane. In this embodiment it may be possible to omit the presence of an ethylbenzene conversion unit if this embodiment is used in a xylenes loop.

In a further embodiment the or each molecular sieve membrane unit comprises two or more alternating zones of catalyst and molecular sieve membrane.

The present invention further provides for an ethylbenzene conversion plant comprising:
(a) a molecular sieve membrane unit comprising a molecular sieve membrane and
(b) a means for ethylbenzene conversion,
wherein the means for ethylbenzene conversion is downstream of the membrane unit.

In one aspect of the present invention a molecular sieve membrane is used to selectively separate ethylbenzene with a small amount of paraxylene from a paraxylene depleted feedstream as is typically found after for example a paraxylene separation process. In this aspect the molecular sieve membrane unit is located upstream of an ethylbenzene conversion unit. The feed to the conversion unit is enriched in ethylbenzene and improves the efficiency of the ethylbenzene conversion process in this unit. The output from this unit is significantly depleted in ethylbenzene and enriched in paraxylene and may pass into a conventional paraxylene isomerisation unit along with the retentate from the molecular sieve membrane *unit.* In such a process the paraxylene isomerisation unit is required to convert lower levels of ethylbenzene and therefore may be operated at lower temperatures and may in fact be a liquid phase isomerisation unit which has no ethylbenzene conversion activity. The overall effect of this use of the molecular sieve membrane is to enhance the conversion of ethylbenzene to useful xylenes and to significantly reduce the xylene losses which usually occur due to the use of high temperature isomerisation units such as ISOMAR™ or MHTI ™. À further modification of this aspect of the present invention is to include a catalytic function into the molecular sieve membrane unit. This catalytic function may be for ethylbenzene conversion and may be located within the membrane itself. This catalytic function may advantageously be located proximate to the membrane on the permeate side of the molecular sieve membrane. The function of this catalyst is to catalyse the conversion of ethylbenzene to xylenes. The effect of this is to deplete or maintain at relatively low levels the concentration of ethylbenzene on the permeate side of the membrane; in doing so this improves the concentration gradient across the membrane which acts as an increased driving force for the transport of ethylbenzene across the membrane. If the ethylbenzene conversion catalyst in the molecular sieve membrane unit is particularly efficient there may be no need for the ethylbenzene conversion unit which is located downstream of the molecular sieve membrane unit. In a further embodiment a second molecular sieve membrane unit for selective paraxylene separation or for selective paraxylene separation and isomerisation, may be located downstream of the paraxylene isomerisation unit. The permeate stream from xylenes isomerisation unit or the second molecular sieve membrane unit if present may be fractionated to remove materials boiling below and above the boiling point of xylenes e.g. benzene, toluene and C9+ aromatics and then transferred to the paraxylene recovery unit. Optionally, the retentate stream may be combined with the permeate stream and the combined streams fractionated and transferred to the paraxylene recovery unit for recovery of a paraxylene rich stream.

The molecular sieve membrane unit apart from comprising a molecular sieve membrane may comprise additional features which are necessary to operate the membrane unit in the chosen application. Such additional features will depend to some extent on the exact nature of the chosen application and the modality of operation of the molecular sieve membrane unit.

In separation processes molecular sieve membranes especially zeolite membranes can be operated in a variety of modes depending on the phase (i.e. gas or liquid), composition and pressure of the feed. Two of these modes of operation are pervaporation and perstraction. In the pervaporation mode, the feed is in the liquid: phase and molecules permeating through the membrane are transported in the gas phase from the permeate side. In the perstraction mode molecules permeating the molecular sieve membrane are transported away from the permeate side by a flowing sweep stream. The flowing sweep stream can be in either the liquid or gas phase. When the feed and sweep stream are both in the gas phase, the mode of operation is referred to as gas phase perstraction. The present invention applies to these as well as other possible modes of operating a molecular sieve membrane in a separation process. The preferred mode of operation is gas phase perstraction.

Molecular sieve membranes may also be used in a catalytic membrane reactor. A catalytic membrane reactor comprises, in addition to the molecular sieve membrane, a catalyst within the membrane or catalyst proximate to the membrane or both, or the membrane may itself be catalytically active. A catalytic membrane with a catalyst proximate to the membrane is referred to as a permselective wall membrane reactor.

In the case where the molecular sieve membrane unit is being used in the gas phase perstraction of ethylbenzene or paraxylene the molecular sieve membrane unit may comprise additional features for the application of a sweep stream to the permeate side of the membrane, for the application of a purge stream to the retentate side of the membrane, means of providing and maintaining a negative pressure differential across the membrane, heating means for the unit, means for pre-heating sweep and/or purge streams and means for the separation of sweep and/or purge streams from permeate and retentate streams respectively. When the molecular sieve unit is being used in conjunction with an isomerisation unit some or all of these additional features may be shared with such a unit. Some of the features may also be shared with other additional upstream or down stream process units. The exact nature and amount of sharing will in part be dependant on economic factors which make it sensible to utilise the additional feature for both parts of the process e.g. stream heaters, heat exchangers and compressors etc. Ideally the molecular sieve membrane is incorporated in a module as described below for inclusion in the molecular sieve membrane unit.

When the molecular sieve membrane unit comprises a molecular sieve membrane or a permselective wall membrane reactor, and is used in conjunction with a paraxylene isomerisation unit, and is located down stream of the isomerisation unit it is advantageous to maintain the molecular sieve membrane unit at a lower temperature than that of the isomerisation unit. Preferably it is maintained at between 10 to 38°C (50 to 100°F) below the temperature of the isomerisation unit. (Rusty doing calculations). This is advantageous because it allows for optimum membrane unit catalyst life and membrane selectivity.

The paraxylene recovery unit when present uses separation technology to produce a paraxylene enriched stream and a paraxylene depleted stream. Such separation technology includes for example the known processes of fractional crystallisation, or selective adsorption using for example molecular sieve adsorbers. The paraxylene recovery unit may therefore be a fractional crystallisation unit which utilises the difference in freezing points between ethylbenzene, ortho-, meta- and paraxylene or it may be a selective adsorption unit which commonly utilises zeolite materials to selectively adsorb paraxylene from C₈ aromatics streams; the adsorbed paraxylene is recovered after desorbing from the zeolite. The paraxylene recovery unit may also be a combination of such separation units, or may incorporate other less commonly used techniques such as fractional distillation.

Fractional crystallisation units are well known in the art and are described for example in US Patent 4 120 911. Commercially available processes include the crystallisation Isofining process, direct contact CO₂ crystallisers, scraped drum crystallisers, and continuous countercurrent crystallisation processes. The crystalliser may operate for example in the manner described in Machetl et. al. US 3 662 013. Commercial fractional crystallisation processes typically recover about 60% to 68% of the paraxylene from the feed to the paraxylene recovery unit when this feed is an equilibrium or near equilibrium mixture of xylenes and ethylbenzene. The reason for this is that they are limited by the formation of a eutectic between paraxylene and metaxylene. However the actual recovery depends on the composition of the feed with higher recoveries possible when the paraxylene content of the feed is higher than the xylenes equilibrium content.

Selective adsorption units are also well known in the art and are described for example in US 3 706 812, US 3 732 325, US 4 886 929, and references cited therein, the disclosures of which are hereby incorporated by reference. Commercially available processes include UOP PAREX™, and IFP-Chevron ELUXYL™ processes. Commercial zeolite selective adsorption processes may recover higher levels of paraxylene than fractional crystallisation processes; typically they recover over 90% or more typically over 95% of the paraxylene from the feed to the paraxylene recovery unit.

The paraxylene recovery unit produces a paraxylene enriched stream that usually comprises over 99% and may even be as high as 99.9 % paraxylene. The exact amount depends on the process used and the design and operating conditions of the specific plant. The balance in this stream being ethylbenzene, ortho-,and metaxylene, toluene, and C₉ aromatics, paraffin's, naphthenes and possibly small amounts of other materials. The paraxylene recovery also produces a paraxylene depleted stream containing the balance of ethylbenzene, ortho-, and metaxylene, toluene, C₉ aromatics, paraffins, etc. along with any paraxylene fed to the paraxylene recovery unit that is not removed in the paraxylene rich stream. It is this paraxylene depleted stream which is then fed to the molecular sieve membrane unit.

The C₈ aromatics stream which may be used as feed for the paraxylene separation unit when present or to the molecular sieve membrane unit when there is no paraxylene separation unit, may come from a variety of sources in the petrochemical plant. One possible source is from naphtha reforming. Examples of such processes include Exxon POWERFORMING™, UOP Platforming™, IFP Aromizing™. Another possible source is a pyrolysis gasoline C₈ stream from steam cracking processes. Such a stream has a relatively high proportion of ethylbenzene. A further possible source is the UOP Cyclar process for conversion of C₃/C₄ hydrocarbon streams to aromatics (see for example US5,258,563, the disclosure of which is hereby incorporated by reference). A further possible source is from toluene disproportionation and/or C₉ aromatics transalkylation. Examples of such processes include UOP TATORAY™, TORAY TAC9™. Mobil Selective Toluene Disproportionation™ (MSTDP), Mobil Toluene Disproportionation™ (MTDP), IFP Xylenes PLUS™ and FINA T2BX™. There are other possible sources of C₈ aromatics streams. The source of C₈ aromatics stream for the process of the present invention is not critical and may be a single stream or may be a combination of streams from any of the above processes.

The ethylbenzene conversion unit may be identical to a conventional paraxylene isomerisation unit which incorporates ethylbenzene conversion as described below. The unit may be operated under conditions which are significantly different to those used for conventional paraxylene isomerisation. In particular it may be operated with the same catalysts but under conditions which are optimised for the specific ethylbenzene conversion reaction required e.g. conversion to benzene and or xylenes.

The paraxylenes isomerisation unit may be any of the well known units in the art such as those described in US 4 236 996, US 4 163 028, US 4 188 282, US 4 224 141, US 4 218 573, US 4 236 996, US 4 899 011, US 3 856 872 and Re. 30,157, the disclosures of which are hereby incorporated by reference.

The isomerisation catalyst may be any of the well known catalysts for isomerisation units in the art. There are primarily two types of catalyst system which are used in isomerisation units. The choice of catalyst has an impact on the overall yield and structure of the aromatics complex and also on the plant design and economics. The first type of catalyst is designed to convert ethylbenzene to xylenes and to isomerise the paraxylene depleted feed stock to a near equilibrium xylene composition. This type of catalyst system is generally the choice for aromatics producers whose objective is to maximise para and ortho-xylene production from a fixed quantity of feed stock. A second catalyst system is also designed to isomerise the para xylene depleted feed stock; however rather than converting ethylbenzene to xylenes, this catalyst system dealkylates the ethylbenzene to produce benzene. This catalyst system is often employed when the benzene requirements are high relative to ortho and para xylene production or when feed stock availability is not a limiting factor.

Examples of processes and catalyst systems which include the capability of converting ethylbenzene to benzene are the Mobil MHTI (Mobil High Temperature Isomerisation) process and catalyst (see for example US 3 856 871 and US 4 638 105, the disclosures of which are hereby incorporated by reference), the Mobil MHAI (Mobil High Activity Isomerisation) process and catalyst, the AMOCO AMSAC process and catalyst and the UOP ISOMAR™ I-100 process and catalyst.

Examples of processes and catalyst systems which include the capability of converting ethylbenzene to xylenes are the IFP/ENGELHARD Octafining and Octafining II processes and catalyst, and the UOP ISOMAR™ I-9 process and catalyst. Other processes include catalysts capable of converting ethylbenzene to C₁₀ aromatics via alkylation or transalkylation.

Isomerization units typically use a zeolite or mordenite type catalyst. Isomerization catalysts known to promote conversion of ortho- and metaxylene to paraxylene include metal promoted zeolites such as for example Pt Promoted ZSM-5, Pt promoted Mordenite and metal promoted borosilicates etc. Commercial examples are Mobil MHAI and ISOMAR™ 1-9 catalyst.

The isomerization reactor is arranged and effective to isomerise ortho- and metaxylene to paraxylene at these conditions and also advantageously to convert ethylbenzene to benzene and/or xylenes. Desirably it may be a liquid phase isomerisation unit operated at low temperatures when little or no ethylbenzene conversion takes place. The term "arranged and effective" is used in this application to denote the conditions in a process unit as described in this specification, such as the temperatures, pressures, space velocities, reaction time, other reactants, and any other process conditions necessary to achieve the desired reaction, conversion or separation that is the normal function of that process unit. As the ethylbenzene is converted to xylenes in the ethylbenzene converter the paraxylenes isomerisation unit may be operated under optimised conditions for xylenes isomerisation as there may be no need to convert ethylbenzene in this unit if it is fully converted in the ethylbenzene conversion unit.

Operating temperatures are typically in the range of 204 to 482°C (400 to 900°F) and pressures in the range of 1.724 to 34.474 bar (25 to 500 PSIG). The weight hourly space velocity (WHSV) based on hydrocarbon feed typically ranges from 0.5 to 20. Most isomerization catalyst systems require a source of hydrogen which can be introduced to the isomerization reactor to promote the isomerization reaction that converts ortho- and metaxylene to paraxylene, to assist in the conversion of ethylbenzene to benzene and or xylenes and assists also in the prevention of coking of the isomerisation catalyst.

Even if an isomerisation or ethylbenzene conversion catalyst is used which does not require hydrogen it is preferred that hydrogen is present in the process of the present invention when the molecular sieve membrane is a zeolite membrane and the zeolite membrane is susceptible to flux decay.

It has been discovered that operating molecular sieve membranes and molecular sieve membrane composites in the presence of hydrogen substantially reduces flux decay. Stated differently, it has been discovered that in separation processes an amount of hydrogen can be added in order to maintain transport flux at a predetermined acceptable level. Flux decay reduction is achieved when hydrogen is present in the feed stream, the permeate, or the optional sweep stream when present, or in any combination of feed stream, permeate, and optional sweep stream. When molecular sieve membranes and molecular sieve membrane composites are operated without the presence of hydrogen while using a hydrocarbon feed, the flux through the membrane is observed to decay in an approximately exponential fashion with time.

In the absence of hydrogen, permeate flux, F, through the molecular sieve membrane at any particular time, t, can be approximately determined from the flux at some arbitrary earlier time, F₀ by the use of a rate constant, K, according to the following relationship:${\text{F=F}}_{\text{0}} {\text{e}}^{\text{-Kt}} \text{.}$

The units of flux used herein are Kg/M²-day. This relationship describes an exponential decay of flux with time. Using this relationship, a lifetime for the flux, τ , can be defined as the length of time for the permeate flux to decay to 1/e of its original value. τ can be calculated from the relationship$\text{τ=1/K.}$

Similarly, a flux half life, t_{1/2}, is defined as the time in which the initial flux decays to half its original value. The half life is determined from the relationship${\text{t}}_{\text{1/2}} \text{=τ(ln 1/2).}$

The rate of flux decay, K, increases with operating temperature and depends on the molecular composition of the feed stock and membrane composition. This approximately exponential decay of flux with time is especially troublesome during high temperature separation processes with hydrocarbon feeds. It is especially advantageous to operate the separation process at high temperatures because the diffusion coefficients of typical permeates increases with temperature. Additionally, in the case of membranes used as components in a catalytic membrane reactor, high temperatures may be required because many catalytic processes operate only at high temperatures. However, the half life for the flux decreases dramatically as the operating temperature is increased.

An example where transport flux reduction would be especially troublesome is the separation of paraxylene and/or ethylbenzene permeate from a feed stream comprising a mixture of xylenes. Transport flux decay would quickly reduce the paraxylene and/or ethylbenzene yield in the preferred separation temperature range of 170°C to 500 °C, and flux decay has been routinely observed with permselective molecular sieve membranes and molecular sieve membrane composites fabricated on alumina and steel supports. MFI zeolite membranes and MFI zeolite membrane composites that were permselective for paraxylene and/or ethylbenzene were found to have a flux half life at 225 °C of 10 to 10000 times less than at room temperature when operated with an argon sweep removing the permeate from a mixed A8 aromatic feed. However, while separating xylenes at 300 °C, first with an inert sweep gas such as argon or nitrogen and then adding hydrogen to the sweep, transport flux was increased and remained stable when hydrogen was present, whereas it decayed when hydrogen was absent

While the flux decay prevention and remediation through the presence of hydrogen may be observed in all cases where the feedstream is derived from hydrocarbons, the rate of change of transport flux in time depends on separation and separation/catalysis process conditions such as temperature, pressure, transmembrane pressure drop or differential, and space velocity. The rate of change also will depend on feed composition, membrane and membrane composite composition and structure, hydrogen partial pressure, the ratio of the amount of hydrogen present to the amount of feed, and other similar parameters. The exact rate of flux decay varies with choice of membrane and also increased slightly with feed pressure. Feed pressure of the mixed A8 aromatic feed (xylenes and ethylbenzene) varied from 1.013 bar (1 atmosphere) to 15.199 bar (15 atmospheres) in these experiments and sweep pressure was maintained equal to or 2.027 to 10.133 bar (2 to 10 atmospheres) below the feed pressure. At temperatures approaching ∼400 °C the half life of the flux was in general less than ∼2 days when hydrogen was not present during separations. This rapid rate of flux decay is a problem for any practical higher temperature application of molecular sieve membranes. Higher temperature operation is desirable because permselective transport increases with temperature. For example, in a preferred embodiment such as paraxylene and/or ethylbenzene separation the invention is practised at temperatures above 225 °C and preferably at temperatures above 300 °C.

Reductions in flux decay occur when the separation is conducted in the presence of hydrogen in an amount of at least about 0.1 mole percent of the total molecules in the feed and sweep. At temperatures greater than 225 °C at least 1 mole percent hydrogen must be present, although further reductions in flux decay will be observed when there is at least 10 mole percent hydrogen in the total number of molecules in the feed and sweep. At temperatures greater than 300 °C at least 20 mole percent hydrogen should be in the total number of molecules in the feed and sweep although further reductions in flux decay will be observed when about 30 to about 60 mole percent hydrogen is present In the preferred embodiment of the present invention in which an isomerisation catalyst is used in combination with a molecular sieve membrane to provide enhanced production of paraxylene the hydrogen content of the feed and sweep must be sufficient to ensure optimum catalyst performance. When considering the hydrogen requirements for both flux decay and for catalyst performance the level of hydrogen used is dictated by the most critical of these factors. For example if a catalyst is used which does not require hydrogen then the hydrogen requirements for flux decay prevail however if the catalyst requires a particularly high level of hydrogen e.g. 75 mole percent or even 90 mole percent, then the hydrogen requirements for the catalyst performance are likely to meet the hydrogen requirements for prevention of flux decay.

The addition of hydrogen to separation and combinations of separation and catalytic reactions reduces flux decay in cases where hydrogen is not present in sufficient quantity initially. Hydrogen can be added to either the feed or sweep if one is employed in the process. However, it should be noted that hydrogen readily diffuses through molecular sieve membranes, and therefore contacts both faces of the molecular sieve membrane regardless of whether it is introduced on the feed stream or permeate side of the membrane.

In one aspect of the present invention the molecular sieve membrane unit is used to selectively separate paraxylene and/or ethylbenzene from a stream which comprises ethylbenzene and an equilibrium or near equilibrium mixture of xylenes.

In this aspect the molecular sieve membrane unit may be located downstream of an isomerisation unit and does not have an isomerisation catalyst in combination with the membrane.

In a further aspect of the present invention, the molecular sieve membrane unit for ethylbenzene separation utilises an isomerisation catalyst in combination with the membrane to convert ethylbenzene to xylenes and /or benzene.

In a further embodiment of the present invention the molecular sieve membrane in the molecular sieve membrane unit downstream of the paraxylene isomerisation unit may itself be rendered catalytically active for the isomerisation reaction or an appropriate isomerisation catalyst may be located proximate to the membrane. By proximate to the membrane is meant that the catalyst is arranged and effective to isomerise the ortho- and/or metaxylene and/or ethylbenzene in the material in the molecular sieve membrane unit but upstream of the zeolite membrane to produce paraxylene. The exact amount of paraxylene which is required to be produced by the isomerisation process in the molecular sieve membrane unit depends in part on the properties of the molecular sieve membrane used. If the membrane for example has high flux and/or high selectivity for paraxylene then it may be possible or even desirable for the isomerisation reaction to produce and maintain paraxylene at a none equilibrium concentration compared to its concentration in an equilibrium xylene mixture whilst the membrane selectively removes paraxylene from the upstream material and into the permeate. However the isomerisation catalyst in the molecular sieve membrane unit should ideally be arranged and effective to produce and maintain paraxylene, upstream of the membrane and inside the molecular sieve membrane unit, at 50 % or more, preferably 80 % or more, and most preferably 90 % or more of the paraxylene equilibrium concentration whilst the membrane selectively removes paraxylene from the upstream side of the membrane and into the permeate. Depending on membrane properties it may be desirable and preferable to maintain the paraxylene concentration at or near to equilibrium for xylenes isomerisation whilst the membrane selectively removes paraxylene from the retentate into the permeate. Thus the isomerisation catalyst causes the ortho- and metaxylene to convert to paraxylene and the paraxylene selectively permeates through the molecular sieve membrane to be produced as a permeate stream. Ortho- and metaxylene less readily pass through the molecular sieve membrane and tend to stay on the upstream side in the retentate stream where they can be further isomerised. The permeate stream from xylenes isomerisation unit may be fractionated to remove materials boiling below and above the boiling point of xylenes e.g. benzene, toluene and C9+ aromatics and then transferred to the paraxylene recovery unit. If the molecular sieve membrane unit is particularly efficient at isomerisation and separation there may theoretically be no retentate stream as there would be no paraxylene depleted stream to reject. In practice there will however likely be impurities and heavier aromatic compounds such as C₉ aromatics which remain in the retentate stream and must be purged from the molecular sieve membrane unit for further treatment. Thus in the molecular sieve membrane unit there is a dynamic and coupled process of isomerisation and separation of xylenes. If the catalytic function is also capable of converting ethylbenzene to benzene or xylenes then any ethylbenzene which enters into the retentate stream of the unit is also involved in this dynamic process with the resulting xylenes entering into the xylenes isomerisation reactions or the resulting benzene passing through the membrane into the permeate stream. In this aspect the molecular sieve membrane unit may be downstream of an isomerisation unit or may be used in place of an isomerisation unit.

The molecular sieve membrane used in the molecular sieve membrane unit may be any molecular sieve material in the form of membrane which is permselective for ethylbenzene compared to other C8 aromatics such as the xylenes especially meta and orthoxylene, when used to produce a permeate rich in ethylbenzene for the ethylbenzene conversion unit. When used in the separation of paraxylene from a mixture of aromatics as is the case when a molecular sieve membrane unit is utilised downstream of the xylene isomerisation unit it is preferred that the membrane has a binary selectivity for paraxylene to orthoxylene or metaxylene of at least 2:1. Examples of such molecular sieve materials include controlled pore amorphous materials such as MCM-41 and zeolite molecular sieves. It is preferred that the molecular sieve membrane is a zeolite membrane.

Examples of zeolite membranes which may be used in zeolite membrane units for the present invention are described in the following documents. U. S. Patent 5,110,478, the disclosure of which is hereby incorporated by reference, describes the direct synthesis of zeolite membranes. The membranes produced in accordance with the teachings of U.S. Patent 5,110,478 were discussed in "Synthesis and Characterisation of a Pure Zeolite Membrane," J. G. Tsikoyiannis and W. Haag, Zeolites (VOI. 12, p. 126., 1992). Such membranes are free standing and are not affixed or attached as layers to any supports. Zeolite membranes have also been grown on supports. See e.g. "High temperature stainless steel supported zeolite (MFI) membranes: Preparation, Module, Construction and Permeation Experiments," E. R. Geus, H. vanBekkum, J. A. Moulyin, Microporous Materials, Vol. 1, p. 137, 1993; Netherlands Patent Application 91011048; European Patent Application 91309239.1 and U.S. Patent 4,099,692, the disclosures of which are hereby incorporated by reference. Other literature describing supported inorganic crystalline molecular sieve layers includes U.S. Patent No. 4,699,892; J. C. Jansen et al, Proceedings of 9th International Zeolite Conference 1992 (in which lateral and axial orientations of the crystals with respect to the support surface are described), J. Shi et al, Synthesis of Self-supporting Zeolite Films, 15th Annual Meeting of the British Zeolite Association, 1992, Poster Presentation (in which oriented Gmelinite crystal layers are described); and S. Feng et al, Nature, 28th April, 1994, p 834 (which discloses an oriented zeolite X analogue layer), the disclosures of which are hereby incorporated by reference.

Further examples of zeolite membranes which may be used in zeolite membrane units for the present invention are described in the following documents; International Application WO 94/25151, USSN 267760 filed 8th July 1994, PCT US95/08512, PCT US95/08514, PCT US95/08513, PCT EP95/02704 and WO94/01209, the disclosures of which are hereby incorporated by reference. In our earlier International Application WO 94/25151 we have described a supported inorganic layer comprising optionally contiguous particles of a crystalline molecular sieve, the mean particle size being within the range of from 20 nm to 1 µm. The support is advantageously porous. When the pores of the support are covered to the extent that they are effectively closed, and the support is continuous, a molecular sieve membrane results; such membranes have the advantage that they may perform catalysis and separation simultaneously if desired. Preferred zeolite membranes are those which are prepared by the Inverted In-Situ-Crystallisation (I-ISC) process, or by using a GEL layer and a Low Alkaline synthesis solution using the Inverted In-Situ-Crystallisation process (GEL-LAI-ISC),or by using a Seeding Layer and a Low-Alkaline-synthesis solution using the Inverted In-Situ Crystallisation (S-LAI-ISC). These processes are described in USSN 267760 filed 8th July 1994, PCT US95/08512, PCT US95/08514, PCT US95/08513 and PCT EP95/02704. Zeolite compositions fabricated using the above described LAI-ISC, GEL-LAI-ISC, or S-LAI-ISC techniques can have dense zeolite layers in which the zeolite crystals are intergrown such that non-selective permeation pathways in these as-synthesised zeolite layers are virtually nonexistent. The zeolite membranes described above may be incorporated into the zeolite membrane unit in the form of a module such as that described in WO94/01209. It is envisaged that the zeolite membrane unit will contain at least one zeolite membrane which may or may not be catalytically active. If the membrane is not catalytically active for the desired process a suitable catalyst may be used in combination with the membrane. This catalyst may be located on the upstream side of the membrane or the downstream side of the membrane depending on the process and the nature and purpose of the catalyst. In one embodiment one or more membranes may be arranged with one or more catalysts to provide alternating membrane and catalyst regions in the zeolite membrane unit. In this arrangement the feedstream to the unit may for example pass through a membrane region with the retentate flowing to a catalyst containing region and then through a second membrane region to a second catalyst region. The exact number of membrane and catalyst regions will depend on the nature of the separations and catalyst processes desired. The separation and catalyst process may be substantially the same for each combination of catalyst and membrane or may be different.

It should be understood that two or more zeolite membrane units with or without isomerisation catalyst in close proximity to the zeolite membrane in each unit may be used in the processes of the present invention. Reference to zeolite membrane unit in this specification should also be taken to include embodiments where two or more zeolite membrane units may be used in sequence to each other with or without any further intervening processes or process units.

The zeolite membranes described above may be incorporated into the molecular sieve membrane unit in the form of a module such as that described in WO94/01209 the disclosure of which is incorporated by reference. In the context of the present invention the term module signifies the combination of a molecular sieve membrane which may or may not be deposited on a substrate supported within a housing which has all the means and additional features required to effectively include the membrane into the molecular sieve unit. These additional means and features include valves and pipework which is necessary to attach the module to the other components of the molecular sieve membrane unit. The module may therefor comprise some or all of those features which are required to operate a molecular sieve membrane unit.

The module may comprise a housing preferably made out of metal such as stainless steel which can withstand the temperatures, pressures and chemical environment to which the membrane unit is exposed in operation. The module will contain a membrane element which comprises the molecular sieve membrane either self-supporting or on a suitable support. It is preferred that, for robustness, the molecular sieve membrane is deposited on a suitable support. Suitable supports are inorganic supports which are able to withstand the operating environment of the molecular sieve membrane unit and include stainless steel and ceramic supports. Examples of suitable supports are provided in the references cited above for zeolite membranes. The exact method of incorporating the membrane element into the module will depend on the nature of the materials in the module housing and element. If the housing is stainless steel one method of incorporation is as described in WO94/01209 the disclosure of which is incorporated by reference. Another possible arrangement is to incorporate a number of membrane elements which comprise zeolite membrane on a porous stainless steel support within a plate and frame arrangement. Such arrangements are well known in the art. If the membrane element comprises a ceramic support for example when it is a ceramic corderite monolith then it may be required that suitable seals are utilised to incorporate the membrane element into a metal housing. Such seals should be capable of withstanding the operating conditions of the membrane unit and are known in the art The module will also have means for introducing the feed to the upstream side of the membrane and means for removing the product from the downstream side of the membrane. On the upstream side the module may be engineered to provide a purge for removing retentate. On the downstream side the module may be engineered to provide a sweep gas to remove product from the downstream side of the module. The module may also be engineered to allow temperature control by means of heating and/or cooling. The module may also contain means such as valving and pressure regulators to provide and control the pressure within the module such that there is either no pressure differential across the membrane or preferably so that there is a pressure differential between the upstream and downstream side of the membrane such that the pressure on the upstream side of the membrane is higher. The feed may be introduced to the module such that the pressure on the upstream side of the membrane is at 1.013 to 35.444 bar (1 to 35 atm), preferably 10.133 to 30.398 bar (10-30 atm) and most preferably 15.199 to 25.331 bar (15 to 25 atm). The average pressure differential between the upstream and downstream sides of the membrane may be 0 to 35.444 bar (0 to 35 atm), preferably 2.027 to 35.444 bar (2 to 35 atm) and most preferably 5.066 to 15.199 bar (5 to 15 atm). The pressure differential is expressed as an average as there may be a reduction in the upstream and downstream pressures as you move along a membrane element within a module depending on how this is engineered. Also located within the module is the isomerisation catalyst which will be on either the upstream side of the membrane for xylenes isomerisation or on the downstream side of the membrane for the ethylbenzene conversion. The membrane element may be engineered or its arrangement within the module may be such so as to allow counter-current operation between feed and sweep within the module or it may be arranged to allow co-current operation between feed and sweep within the module or it may be arranged for cross-flow operation. Thus the membrane element may comprise a multichannel ceramic monolith support in which some of the channels are utilised for introducing the feed to the element and some of the channels re utilised for introducing the sweep to the element. The feeds may be arranged through the use of appropriate flanges and masking to allow co-current, counter-current or cross-flow operation. When such a multichannel monolith is used the isomerisation catalyst for use in combined isomerisation and separation is located within the feed channels of the monolith or if used for ethylbenzene conversion within the sweep channels. Alternatively the catalyst may be located within the monolithic support but on the feedstream side of the membrane or on the sweep side of the membrane depending on the application. When desirable the combination of the retentate stream (with or without purge) with the permeate stream (with or without sweep) may take place within the module itself.

The membrane elements may be included in the module as described in WO94/01209. The membrane elements may also be incorporated into other known designs for membrane separation units such as for example plate and frame membrane modules. Suitable ceramic monolith elements may be constructed according to US 5 221 484, US 5 114 581, US 5 120 576, EP 0 373 215, US 5 108 601, EP 0 396 684, EP 0 310 632 but with the use of a zeolite membrane. Such monolithic tubes may be incorporated into a metal module by the use of techniques known in the art such as for example EP 0 270 051.

As indicated above the fresh feed for the xylene recovery loop may come from a variety of sources in the petrochemical cycle. Fresh feed from, for example, a reformer, which is introduced to the xylene recovery loop is usually fractionated before introduction to the paraxylene separation unit to remove materials boiling below the boiling point of xylenes, and may optionally also be fractionated to remove at least part of the material boiling above the boiling point of xylenes. If lower boiling materials are not removed from the fresh feed, it is introduced to a detoluenizer tower ("DETOL") which removes toluene and lighter materials by distillation. The feed is then introduced to either a xylene rerun tower or splitter. A xylene rerun tower removes C₉₊ aromatics from the feed. A xylene splitter tower in addition removes at least part of the orthoxylene for subsequent recovery as orthoxylene product in an orthoxylene rerun tower. The fresh feed in a xylenes loop is combined with a recycle stream which comes from the xylene isomerisation unit or in the present invention from the zeolite membrane unit. The overhead stream from the xylene rerun tower or splitter is typically a mixture of compounds which includes 0 to 10 wt% non aromatics, 0 to 5 wt% toluene, 5 to 20 wt% ethylbenzene, 0 to 10 wt% C₈ naphthenes, and 70 to 95 wt% xylenes. The exact composition will depend on the fresh feed and the nature of the catalysts used in the isomerisation unit and in the zeolite membrane unit. It should be appreciated that the fresh feed to the xylenes recovery loop could be a combination of two or more feeds such as those discussed above. Thus it could be a combination of a feed from a naphtha reformer with that from a TATORAY™ or MSTDP™ unit.

It should be understood that in the present description when reference is made to a feed to, or material upstream of the membrane, in a zeolite membrane unit being at equilibrium in xylenes this means that it can be a mixture of xylenes which are at the typical respective concentrations for an equilibrium mixture of xylenes as known in the art. In the same context by near equilibrium is meant a composition comprising xylenes in which one or more of the xylenes present are at their none equilibrium concentration with respect to the other xylenes present and includes mixtures where one or more of the xylene isomers are present at a concentration which is greater than their equilibrium concentration. Ideally in such mixtures the paraxylene should be present at 50 % or more, preferably 80 % or more and most preferably at 90 % or more of the paraxylene equilibrium concentration.

Other objects and features of the invention are described in the following detailed description wherein reference is made to the accompanying figures.

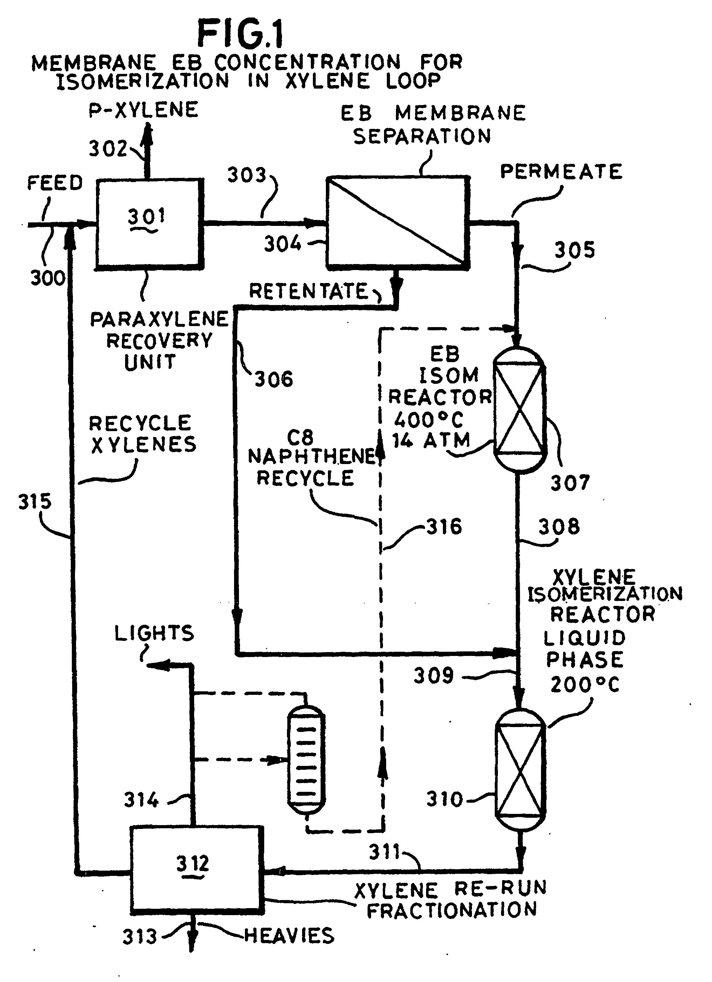

Figure 1 shows a xylene purification loop which utilises an ethylbenzene membrane separation unit upstream of an ethylbenzene conversion unit which is upstream of an xylene isomerisation unit.

In Figure 1 the zeolite membrane unit has as its primary function the separation of ethylbenzene from a paraxylene depleted stream so that this may be passed into an ethylbenzene conversion unit. Thus a feed comprising xylenes and ethylbenzene 300 in combination with recycle xylenes 315 is passed to a paraxylene recovery unit 301. Paraxylene is withdrawn through paraxylene rich stream 302 and ortho- and metaxylene and ethylbenzene are withdrawn through paraxylene poor stream 303. Paraxylene poor stream 303 is introduced to a zeolite membrane unit 304 comprising a zeolite membrane which permits selective permeation of ethylbenzene and possibly paraxylene through the zeolite membrane relative to ortho- and metaxylene. Most of the ethylbenzene and possibly most of the paraxylene is withdrawn from the zeolite membrane unit 304 through permeate stream 305 and most of the ortho- and metaxylene are withdrawn through retentate stream 306. Permeate stream 305 passes into an ethylbenzene conversion unit 307. Ethylbenzene isomerization unit 307 isomerises ethylbenzene to xylenes. The ethylbenzene isomerate is withdrawn through line 308. The retentate 306 and ethylbenzene isomerate 308 are combined to provide a unified feed 309 to the isomerisation unit 310 which may be a liquid phase xylenes isomerisation unit operating at 200°C. The xylenes isomerate passes from the isomerisation unit 310 through line 311 to a xylene re-run fractionation sequence 312 which produces a heavy stream 313, a lights stream 314 and a xylenes recycle 315. The lights stream may be further treated to a fractionation process to produce a C8 naphthene recycle 316 to the ethylbenzene conversion unit 307 (shown as dotted lines in the figure). A possible addition to the process described in Figure 1 is the inclusion of a zeolite membrane unit after the isomerisation unit 310 but before the xylene re-run fractionation sequence 312. This additional zeolite membrane unit would further enrich the stream 311 in paraxylene.

In this embodiment for a given membrane the predicted recovery of ethylbenzene on the permeate side of the membrane is 44 wt% compared to a normal ethylbenzene concentration of 6 to 7 wt%. The ethylbenzene conversion across the ethylbenzene isomerisation unit is 85% which compares favourably with a conventional process where the per pass conversion is 42%. Because a much smaller portion of the xylene loop is subjected to the severe conditions of the ethylbenzene conversion unit there are lower overall xylene losses. Also because the xylenes isomerisation unit conditions are less severe than in a combined ethylbenzene/xylenes isomerisation unit the losses of xylenes in this unit are significantly lower; 1% compared to 3 to 4%. This results in an overall yield for paraxylene for this embodiment of 94.5% compared to 84.5% for the conventional xylene loop without zeolite membrane unit. An additional advantage of this embodiment is that the amount of hydrogen circulation is dramatically reduced as the ethylbenzene conversion is in a separate reactor and hydrogen is not required for the xylenes isomerisation unit. This may result in a significant savings on the cost of operating a paraxylene recovery process according to this embodiment compared to the conventional xylenes loop.

## Claims

1. A process for conversion of ethylbenzene to benzene and/or xylenes which process comprises:
(a) passing at least a portion of a stream comprising ethylbenzene to a zeolite membrane unit comprising a molecular sieve membrane, for a time and under such conditions that at least a portion of the ethylbenzene passes through the molecular sieve membrane to the permeate side of the membrane and
(b) converting the ethylbenzene in at least a portion of said permeate to benzene and/or xylenes.

2. A process as claimed in claim 1 wherein there is present an ethylbenzene conversion catalyst either as part of the molecular sieve membrane or downstream of the molecular sieve membrane but in close proximity to the membrane or both.

3. A process as claimed in claim 1 or claim 2 wherein the stream in step (a) comprises a hydrocarbon feedstream comprising paraxylene and at least one other isomer of xylene, ethylbenzene, or mixtures thereof and which process further comprises:
recovering by means of a paraxylene separation process in a paraxylene recovery unit a portion of said paraxylene from at least a portion of said hydrocarbon feedstream to produce a first stream having a reduced paraxylene content compared to the feed stream and comprising at least a portion of said one or other isomers of xylene, said ethylbenzene, or mixtures thereof; and it is at least a portion of this first stream which is fed to the molecular sieve membrane unit.

4. A process as claimed in any of claims 1 to 3 wherein the ethylbenzene conversion is performed in an ethylbenzene conversion unit which is downstream of the molecular sieve membrane unit and that at least a portion of the permeate from the molecular sieve membrane unit is fed to the ethylbenzene conversion unit.

5. A process as claimed in any of claims 1 to 4 wherein the ethylbenzene conversion unit produces a stream which is depleted in ethylbenzene when compared to the permeate from the molecular sieve membrane unit and which is enriched in ethylbenzene conversion products such as benzene and/or xylenes.

6. A process as claimed in any of claims 1 to 5 wherein the stream from the ethylbenzene conversion unit, optionally combined with the retentate from the molecular sieve membrane unit, is subjected to a xylenes isomerisation process in an isomerisation unit to produce an isomerate stream having an enriched paraxylene content compared to the feed to the isomerisation unit.

7. A process as claimed in claim 6 wherein the isomerate is fed to a paraxylene recovery process.

8. A process as claimed in claim 6 wherein isomerate is fed to a second molecular sieve membrane unit downstream of the isomerisation unit and which membrane unit produces a permeate enriched in paraxylene compared to the isomerate.

9. A process as claimed in claim 8 wherein the second molecular sieve membrane unit further comprises a xylenes isomerisation catalyst.

10. A process as claimed in any of the preceding claims where the or each molecular sieve membrane unit comprises two or more alternating zones of catalyst and membrane.

11. An ethylbenzene conversion plant comprising:
(a) a molecular sieve membrane unit (304) comprising a molecular sieve membrane and
(b) a means for ethylbenzene conversion (307), wherein the means for ethylbenzene conversion is downstream of the membrane.

12. A plant as claimed in claim 11 wherein the means for ethylbenzene conversion (307) is an ethylbenzene conversion catalyst which is part of the molecular sieve membrane (304) or downstream of the molecular sieve membrane but in close proximity to the membrane or both.

13. A process as claimed in claim 1 which comprises a zeolite membrane which has been prepared by either the Low Alklaine Inverted In-Situ Crystallisation (LAI-ISC), Seeded Low Alklaine Inverted In-Situ Crystallisation (S-LAI-ISC) or Gel Low Alklaine Inverted In-Situ Crystallisation (GEL-LAI-ISC) processes.

## Patentansprüche

1. Verfahren zum Umwandeln von Ethylbenzol in Benzol und/oder Xylole, bei dem
(a) mindestens ein Teil eines Ethylbenzol enthaltenden Stroms für eine Zeitdauer und unter solchen Bedingungen zu einer eine Molekularsiebmembran aufweisenden Zeolithmembrananlage geleitet wird, so dass mindestens ein Teil des Ethylbenzols durch die Molekularsiebmembran auf die Permeatseite der Membran gelangt, und
(b) das Ethylbenzol in mindestens einem Teil des Permeats in Benzol und/oder Xylole umgewandelt wird.

2. Verfahren nach Anspruch 1, bei dem ein Ethylbenzol-Umwandlungskatalysator entweder als Teil der Molekularsiebmembran oder der Molekularsiebmembran nachgeordnet, jedoch in enger Nähe zu der Membran, oder in beiden Formen vorhanden ist.

3. Verfahren nach Anspruch 1 oder Anspruch 2, bei dem der Strom in Stufe (a) einen Kohlenwasserstoffeinsatzmaterialstrom enthält, der para-Xylol und mindestens ein anderes Isomer von Xylol, Ethylbenzol oder Mischungen derselben enthält und bei dem:
mittels eines para-Xylolabtrennungsverfahrens in einer para-Xylolrückgewinnungsanlage ein Teil des para-Xylols aus mindestens einem Teil des Kohlenwasserstoffeinsatzmaterialstroms gewonnen wird, um einen ersten Strom zu erzeugen, der einen im Vergleich zu dem Einsatzmaterialstrom verringerten para-Xylolgehalt aufweist und mindestens einen Teil des einen Isomers oder der anderen Isomere von Xylol, das Ethylbenzol oder Mischungen derselben enthält, und mindestens ein Teil dieses ersten Stroms in die Molekularsiebmembrananlage eingespeist wird.

4. Verfahren nach einem der Ansprüche 1 bis 3, bei dem die Ethylbenzolumwandlung in einer Ethylbenzolumwandlungsanlage durchgeführt wird, die der Molekularsiebmembrananlage nachgeordnet ist, und mindestens ein Teil des Permeats von der Molekularsiebmembrananlage in die Ethylbenzolumwandlungsanlage eingespeist wird.

5. Verfahren nach einem der Ansprüche 1 bis 4, bei dem die Ethylbenzolumwandlungsanlage einen Strom erzeugt, der verglichen mit dem Permeat aus der Molekularsiebmembrananlage an Ethylbenzol verarmt und an Ethylbenzolumwandlungsprodukten wie Benzol und/oder Xylolen angereichert ist.

6. Verfahren nach einem der Ansprüche 1 bis 5, bei dem der Strom aus der Ethylbenzolumwandlungsanlage, gegebenenfalls in Kombination mit dem Retentat aus der Molekularsiebmembrananlage, in einer Isomerisierungsanlage einem Isomerisierungverfahren für Xylole unterzogen wird, um einen Isomerrisatstrom zu erzeugen, der einen verglichen mit dem Einsatzmaterial für die Isomerisierungsanlage angereicherten para-Xylolgehalt hat.

7. Verfahren nach Anspruch 6, bei dem das Isomerisat in ein para-Xylolrückgewinnungsverfahren eingespeist wird.

8. Verfahren nach Anspruch 6, bei dem Isomerisat in eine der Isomerisierungsanlage nachgeordnete zweite Molekularsiebmembrananlage eingespeist wird, und bei dem die Membrananlage ein Permeat erzeugt, das verglichen mit dem Isomerisat an para-Xylol angereichert ist.

9. Verfahren nach Anspruch 8, bei dem die zweite Molekularsiebmembrananlage ferner einen Isomerisierungskatalysator, für Xylole aufweist.

10. Verfahren nach einem der vorhergehenden Ansprüche, bei dem die oder jede Molekularsiebmembrananlage zwei oder mehr alternierende Zonen von Katalysator und Membran aufweist.

11. Ethylbenzolumwandlungsanlage, die
(a) eine Molekularsiebmembrananlage (304), die eine Molekularsiebmembran aufweist, und
(b) ein Mittel zur Ethylbenzolumwandlung aufweist, wobei das Mittel zur Ethylbenzolumwandlung der Membran nachgeordnet ist.

12. Anlage nach Anspruch 11, bei der das Mittel zur Ethylbenzolumwandlung (307) ein Ethylbenzolumwandlungskatalysator ist, der Teil der Molekularsiebmembran (304) oder der Molekularsiebmembran nachgeordnet, jedoch in enger Nähe zu der Membran, oder beides ist.

13. Verfahren nach Anspruch 1, das eine Zeolithmembran aufweist, die entweder nach dem Verfahren der invertierten in-situ Kristallisation mit geringem Alkaligehalt (LAI-ISC), invertierten in-situ Kristallisation mit geringem Alkaligehalt und Impfkristallen (S-LAI-ISC) oder den der invertierten in-situ Kristallisation mit geringem Alkaligehalt aus Gel (GEL-LAI-ISC) hergestellt worden ist.

## Revendications

1. Procédé pour la conversion d'éthylbenzène en benzène et/ou xylènes, procédé qui comprend les étapes consistant :
(a) à faire passer au moins une partie d'un courant comprenant de l'éthylbenzène jusqu'à une unité à membrane zéolitique comprenant une membrane d'un tamis moléculaire, pendant un temps et dans des conditions telles qu'au moins une partie de l'éthylbenzène passe à travers la membrane de tamis moléculaire jusqu'à la face de perméat de la membrane, et
(b) à convertir l'éthylbenzène d'au moins une partie dudit perméat en benzène et/ou xylènes.

2. Procédé suivant la revendication 1, dans lequel est présent un catalyseur de conversion d'éthylbenzène en tant que partie de la membrane de tamis moléculaire ou en aval de la membrane de tamis moléculaire mais à proximité immédiate de la membrane ou bien à la fois en tant que partie et en aval mais à proximité immédiate.

3. Procédé suivant la revendication 1 ou la revendication 2, dans lequel le courant dans l'étape (a) comprend un courant d'hydrocarbures d'alimentation comprenant du paraxylène et au moins un autre isomère du xylène, de l'éthylbenzène ou leurs mélanges, procédé qui comprend en outre les étapes consistant :
à recueillir au moyen d'un procédé de séparation de paraxylène dans une unité de récupération de paraxylène une partie dudit paraxylène à partir d'au moins une partie dudit courant d'hydrocarbures d'alimentation pour produire un premier courant ayant une teneur réduite en paraxylène comparativement au courant de charge d'alimentation et comprenant au moins une partie dudit autre isomère de xylène, dudit éthylbenzène ou de leurs mélanges ; et au moins une partie de ce premier courant étant amenée à l'unité à membrane de tamis moléculaire.

4. Procédé suivant l'une quelconque des revendications 1 à 3, dans lequel la conversion d'éthylbenzène est effectuée dans une unité de conversion d'éthylbenzène qui est en aval de l'unité à membrane de tamis moléculaire, au moins une partie du perméat provenant de l'unité à membrane de tamis moléculaire étant amenée à l'unité de conversion d'éthylbenzène.

5. Procédé suivant l'une quelconque des revendications 1 à 4, dans lequel l'unité de conversion d'éthylbenzène produit un courant qui est appauvri en éthylbenzène comparativement au perméat provenant de l'unité à membrane de tamis moléculaire et qui est enrichi en produits de conversion d'éthylbenzène tels que le benzène et/ou les xylènes.

6. Procédé suivant l'une quelconque des revendications 1 à 5, dans lequel le courant provenant de l'unité de conversion d'éthylbenzène, facultativement associé au rétentat provenant de l'unité à membrane de tamis moléculaire, est soumis à un procédé d'isomérisation de xylènes dans une unité d'isomérisation pour produire un courant de produits d'isomérisation ayant une teneur enrichie en paraxylène comparativement à la charge d'alimentation amenée à l'unité d'isomérisation.

7. Procédé suivant la revendication 6, dans lequel le produit d'isomérisation est soumis à un procédé de récupération de paraxylène.

8. Procédé suivant la revendication 6, dans lequel le produit d'isomérisation est amené à une seconde unité à membrane de tamis moléculaire en aval de l'unité d'isomérisation, unité à membrane qui produit un perméat enrichi en paraxylène comparativement au produit d'isomérisation.

9. Procédé suivant la revendication 8, dans lequel la seconde unité à membrane de tamis moléculaire comprend un catalyseur d'isomérisation de xylènes.

10. Procédé suivant l'une quelconque des revendications précédentes, dans lequel la ou chaque unité à membrane de tamis moléculaire comprend deux ou plus de deux zones alternées de catalyseur et de membrane.

11. Installation de conversion d'éthylbenzène comprenant :
(a) une unité à membrane de tamis moléculaire (304) comprenant une membrane de tamis moléculaire, et
(b) un moyen de conversion d'éthylbenzène (307), ledit moyen de conversion d'éthylbenzène étant situé en aval de la membrane.

12. Installation suivant la revendication 11, dans laquelle le moyen de conversion d'éthylbenzène (307) est un catalyseur de conversion d'éthylbenzène qui fait partie de la membrane de tamis moléculaire (304) ou qui est en aval de la membrane de tamis moléculaire mais à proximité immédiate de la membrane ou bien qui, à la fois, en fait partie et est en aval mais à proximité immédiate.

13. Procédé suivant la revendication 1, qui comprend une membrane zéolitique qui a été préparée par le procédé de cristallisation in situ inversé à basse teneur en matière alcaline (LAI-ISC), le procédé de cristallisation in situ inversée à basse teneur en substance alcaline avec ensemencement (S-LAI-ISC) ou le procédé de cristallisation inversée à basse teneur en substance alcaline en présence d'un gel (GEL-LAI-ISC).
